# EUROPEAN PATENT APPLICATION

(11) **EP 4 356 756 A1**
(43) Date of publication of application: **24.04.2024**
(21) Application number: 22825207.8
(22) Date of filing: 03.06.2022
(51) Int. Cl.: A23L 33/10, A23L 5/10, A23L 19/10, A61K 36/9068, A61K 31/12, A61P 19/02

(54) **COMPOSITION FOR PREVENTING, IMPROVING, OR TREATING DEGENERATIVE ARTHRITIS COMPRISING STEAMED GINGER EXTRACT OR 1-DEHYDRO-6-GINGERDIONE ISOLATED THEREFROM AS ACTIVE INGREDIENT**

(30) Priority: 15.06.2021 KR 20210077699
(71) Applicant: Genencell Co., Ltd., Yongin-si, Gyeonggi-do 16950 (KR); UNIVERSITY-INDUSTRY COOPERATION GROUP OF KYUNG HEE UNIVERSITY, Yongin-si, Gyeonggi-do 17104 (KR)
(72) Inventor: KANG, Se Chan, Seongnam-si Gyeonggi-do 13608 (KR); JEONG, Yong Joon, Yongin-si Gyeonggi-do 16950 (KR); KIM, Tae Woo, Ansan-si Gyeonggi-do 15502 (KR); PARK, Jae-Hyun, Seoul 06637 (KR); JEON, Hyelin, Seoul 04420 (KR); PARK, Dae Won, Seoul 01033 (KR); YANG, Yoon Jung, Yeoju-si Gyeonggi-do 12667 (KR); KIM, Eun Jeong, Yongin-si Gyeonggi-do 17095 (KR); KIM, In Hye, Anyang-si Gyeonggi-do 14070 (KR); KANG, Tong Ho, Seoul 04096 (KR); LEE, Yeong-Geun, Suwon-si Gyeonggi-do 16692 (KR); KWON, Jeong Eun, Suwon-si Gyeonggi-do 16705 (KR)
(74) Representative: WSL Patentanwälte Partnerschaft mbB
(86) International application number: PCT/KR2022/007917
(87) International publication number: WO 2022/265281

(57) **Abstract**

The present invention relates to a composition for preventing, improving, or treating degenerative arthritis comprising a steamed ginger extract or 1-dehydro-6-gingerdione isolated therefrom as an active ingredient. The steamed ginger extract according to the present invention or 1-dehydro-6-gingerdione isolated therefrom exhibits an effect of inhibiting inflammatory cytokine expression, cartilage tissue recovery, cartilage production, or cartilage degradation, and inhibits the expression of I-xB or NF-κB protein involved in inflammatory cytokine and MMP production, and thus is expected to be effectively used in functional food compositions, pharmaceutical compositions, or the like for preventing, improving, or treating degenerative arthritis.

## Description

### [Technical Field]

The present invention relates to a composition for preventing, ameliorating or treating degenerative arthritis containing a steamed ginger extract or 1-dehydro-6-gingerdione isolated therefrom as an active ingredient.

This application claims the benefit of priority to Korean Patent Application No. 10-2021-0077699 filed on June 15, 2021, the entire contents of which are incorporated herein by reference.

### [Background Art]

Degenerative arthritis is also called "osteoarthritis" and refers to a condition in which motor function is lost and unstable due to continuous breakage of and damage to the cartilage within the joint by cytokines and cartilage-degrading enzymes expressed in chondrocytes and synovial fluid by physical stimulation of the joint area. Degenerative arthritis is the most common arthritis with a prevalence of approximately 60% in men and approximately 70% in women over the age of 65. Current treatment options for degenerative arthritis are limited and include symptomatic treatment with simple analgesics, non-steroidal anti-inflammatory drugs (NSAIDs) or intra-articular (IA) injectable glucocorticoids and hyaluronic acid (HA) preparations. Non-pharmacological indicators range from physical exercise and weight loss to joint irrigation fluids and ultimately surgical joint replacement.

Nonsteroidal anti-inflammatory drugs (NSAIDs), such as ibuprofen, aspirin, and indomethacin, which are commonly used to treat arthritis, have the advantage of being short-acting, but cause damage to the gastrointestinal tract and thus long-term use thereof is restricted. In addition, there are 22 individually recognized and notified materials relating to joint health, and most thereof are raw materials imported from countries, such as China and India, or raw materials developed and imported from the countries. Therefore, the development of functional joint health materials using Korean raw materials is necessary.

Meanwhile, ginger (*Zingiber officinale* Roscoe) is a perennial tropical plant belonging to the ginger family. There are about 1,400 species of ginger in about 47 genera around the world. Ginger is mainly found in tropical areas such as India and the Malay Archipelago. It has been cultivated in warm regions of Asia since ancient times, but is mainly cultivated in high-temperature areas such as tropical and temperate regions, and Yangha of the genus Ginger, Flower Ginger of the genus Flowering Ginger, and Flower Yangha of the genus Flowering Ginger grow wild in the southern regions of Korea.

Ginger is registered as an edible food in Korea and has been used as an edible ingredient in other countries such as the United States and Europe for a long time. Its main ingredients include 6-gingerol, 8-gingerol, 10-gingerol, 6-shogaol, 6-paradol, 8-gingerol, 1-dehydro-6-gingerdione, and 1-dehydro-8-gingerdione. As food raw materials, rhizomes (healthy), roots, stems, and leaves are registered as edible in the Food Code, and the GLP organization verified safety through single-dose toxicity tests (rodents and non-rodents), three genotoxicity tests (backmutation, chromosomal abnormality, micronucleus test), and 13-week repeated toxicity test (rodents).

In Oriental medicine, as disclosed in the book, Dongui Bogam, the dried ginger rhizome was used as a drug material to treat symptoms of colds such as chills, fever, headache, vomiting, cough, and phlegm, and to treat symptoms of food poisoning such as stomachache and diarrhea. In addition, ginger extracts are known to have antioxidant activity and effects of protecting brain nerves, and relieving gastrointestinal discomfort. Gingerol and shogaol, which are the main ingredients of ginger, have antioxidant, anticancer, antibacterial, and neuroprotective effects, and effects of regulating metabolic syndrome such as diabetes, blood pressure, and cholesterol.

The present inventors attempted to develop functional materials derived from natural products for preventing, ameliorating, or treating degenerative arthritis by analyzing various physiological activities of steamed ginger extracts obtained by steaming ginger.

### [Disclosure]

### [Technical Problem]

As a result of research to develop a material that can reduce the side effects of conventional arthritis treatments and is effective in ameliorating degenerative arthritis, the present inventors prepared a steamed ginger extract, by applying, to ginger, steaming, which is a method generally included in the process of producing red ginseng from ginseng to produce ingredients beneficial to the human body, to improve the absorption rate and to enable long-term storage, and identified that the steamed ginger extract is effective in ameliorating degenerative arthritis. Based thereon, the present invention was completed.

Accordingly, it is one object of the present invention to provide a food composition for preventing or ameliorating degenerative arthritis containing a steamed ginger extract as an active ingredient.

It is another object of the present invention to provide a pharmaceutical composition for preventing or treating degenerative arthritis containing a steamed ginger extract as an active ingredient.

It is another object of the present invention to provide a method for preparing a composition for preventing, ameliorating, or treating degenerative arthritis using a steamed ginger extract.

However, the technical problems to be achieved by the present invention are not limited to the technical problems mentioned above and other technical problems not mentioned herein can be clearly understood by those skilled in the art from the description below.

### [Technical Solution]

In accordance with one aspect of the present invention, the above and other objects can be accomplished by the provision of a food composition for preventing or ameliorating degenerative arthritis containing a steamed ginger extract, 1-dehydro-6-gingerdione isolated therefrom, or a sitologically acceptable salt thereof as an active ingredient.

In accordance with another aspect of the present invention, provided is a pharmaceutical composition for preventing or treating degenerative arthritis containing a steamed ginger extract, 1-dehydro-6-gingerdione isolated therefrom, or a pharmaceutically acceptable salt thereof as an active ingredient.

In an embodiment of the present invention, the food composition may be a health functional food composition, but is not limited thereto.

In another embodiment of the present invention, the steamed ginger extract is obtained by extraction using a solvent selected from the group consisting of water, C₁-C₄ lower alcohol, n-hexane, ethyl acetate, acetone, acetonitrile, n-butylacetate, 1,3-butylene glycol, methylene chloride and a mixture thereof, but is not limited thereto.

In another embodiment of the present invention, the steamed ginger extract contains at least one selected from the group consisting of 1-dehydro-6-gingerdione, 6-gingerol, 8-gingerol, 10-gingerol, 6-shogaol, 6-paradol, 8-paradol, 1-dehydro-8-gingerdione, a sitologically acceptable salt thereof, and a pharmaceutically acceptable salt thereof, but is not limited thereto.

In another embodiment of the present invention, the 1-dehydro-6-gingerdione may be present in an amount of 0.001% to 10% by weight based on the dry weight of the steamed ginger extract, but is not limited thereto.

In another embodiment of the present invention, the composition may reduce expression of at least one selected from the group consisting of IL-1β, TNF-α, IFN-γ, and prostaglandin E₂ (PGE₂), but is not limited thereto.

In another embodiment of the present invention, the composition may have an activity of restoring cartilage tissue, creating cartilage, or inhibiting cartilage decomposition, but is not limited thereto.

In another embodiment of the present invention, the composition may reduce expression of I-κB or nuclear factor (NF)-κB protein, but is not limited thereto.

In accordance with another aspect of the present invention, provided is a method of treating or ameliorating degenerative arthritis including administering a composition containing a steamed ginger extract, 1-dehydro-6-gingerdione isolated therefrom, or a pharmaceutically or sitologically acceptable salt thereof to a subject in need thereof.

In accordance with another aspect of the present invention, provided is the use of a composition containing a steamed ginger extract, 1-dehydro-6-gingerdione isolated therefrom, or a pharmaceutically or sitologically acceptable salt thereof for the prevention, amelioration or treatment of degenerative arthritis.

In accordance with another aspect of the present invention, provided is the use of a composition containing a steamed ginger extract, 1-dehydro-6-gingerdione isolated therefrom, or a pharmaceutically or sitologically acceptable salt thereof for the preparation of drugs for preventing, ameliorating or treating degenerative arthritis.

In accordance with another aspect of the present invention, provided is a method for preparing a composition for preventing, ameliorating, or treating degenerative arthritis containing a steamed ginger extract, the method including:
(a) washing ginger and steaming the ginger at 85 to 105°C for 1 to 3 hours;
(b) drying the steamed ginger at 40 to 60°C for 30 to 50 hours;
(c) extracting the dried steamed ginger in a solvent at 75 to 95°C for 10 to 20 hours;
(d) filtering the extracted steamed ginger, followed by concentration under reduced pressure at a temperature of 10 to 65°C; and
(e) filtering the steamed ginger extract to prepare a steamed ginger extract powder.

### [Advantageous effects]

The steamed ginger extract or the 1-dehydro-6-gingerdione isolated therefrom according to the present invention exhibits the effects of suppressing inflammatory cytokine expression, of restoring cartilage tissue, of creating cartilage, of inhibiting cartilage degradation, and of inhibiting the expression of I-κB or NF-xB protein involved in the production of inflammatory cytokines and MMPs, thus being useful for functional food compositions, pharmaceutical compositions, and the like for preventing, ameliorating, or treating degenerative arthritis.

### [Description of Drawings]

FIG. 1 is a diagram showing changes in 1-dehydro-6-gingerdione of a ginger extract and a steamed ginger extract according to one embodiment of the present invention.
FIG. 2A illustrates the effects of treatment with the steamed ginger extract according to the embodiment of the present invention on cytotoxicity (left) and NO production inhibition (right).
FIG. 2B illustrates the effect of treatment with active ingredients of the steamed ginger extract according to one embodiment of the present invention on cytotoxicity.
FIG. 2C illustrates the change in NO production by treatment with active ingredients of the steamed ginger extract according to one embodiment of the present invention.
FIG. 3A illustrates changes in mRNA expression of IL-1β by treatment with the steamed ginger extract and 1-dehydro-6-gingerdione according to one embodiment of the present invention.
FIG. 3B illustrates the change in protein expression of IL-1β by treatment with the steamed ginger extract and 1-dehydro-6-gingerdione according to one embodiment of the present invention.
FIG. 4 is a schematic diagram illustrating a process of inducing a degenerative arthritis animal model and administering the steamed ginger extract according to an embodiment of the present invention to the animal model.
FIG. 5A is a micro-CT image showing the change in cartilage tissue structure upon administration of the steamed ginger extract to the degenerative arthritis animal model according to one embodiment of the present invention.
FIG. 5B is a diagram showing quantified morphological indicators of cartilage tissue upon administration of the steamed ginger extract to the degenerative arthritis animal model according to an embodiment of the present invention.
FIG. 6 shows changes in the expression of IL-1β, TNF-α, and PGE₂ in the blood of the degenerative arthritis animal model according to the embodiment of the present invention upon administration of the steamed ginger extract.
FIG. 7 illustrates changes in the expression of IL-1β, IFN-γ, and TNF-α in cartilage of the degenerative arthritis animal model according to the embodiment of the present invention upon administration of the steamed ginger extract to the model.
FIG. 8 illustrates changes in the expression of TIMP1, TIMP2, MMP-9, and COL-2 in cartilage of the degenerative arthritis animal model according to the embodiment of the present invention upon administration of the steamed ginger extract to the model.
FIG. 9 illustrates changes in the expression of I-κB and NF-κB proteins in cartilage of the degenerative arthritis animal model according to the embodiment of the present invention upon administration of the steamed ginger extract to the model.

### [Best Mode]

In one embodiment of the present invention, a steamed ginger extract was prepared by steaming ginger (see Example 1).

In another embodiment of the present invention, among 6-shogaol, 6-gingerol, and 1-dehydro-6-gingerdione isolated from the steamed ginger extract, 1-dehydro-6-gingerdione was present in a significantly increased amount, compared to the ginger extract and thus was selected as a marker component (see Example 2).

It can be seen from an experimental example of the present invention that the steamed ginger extract and 1-dehydro-6-gingerdione isolated therefrom exhibited low cytotoxicity and high NO production and IL-1β expression inhibitory effects *in vitro* (See Experimental Example 1).

In another experimental example of the present invention, the steamed ginger extract has effects of reducing expression of inflammatory cytokines, restoring cartilage tissue, creating cartilage, inhibiting cartilage decomposition, and inhibiting the expression of I-κB or NF-κB proteins involved in the production of inflammatory cytokines and MMPs in the blood or cartilage tissue of an animal model with degenerative arthritis induced with MIA (mono-iodoacetate) (see Experimental Example 2).

Therefore, the present invention provides a food composition for preventing or ameliorating degenerative arthritis containing a steamed ginger extract, 1-dehydro-6-gingerdione isolated therefrom, or a sitologically acceptable salt thereof as an active ingredient.

As used herein, the term "steaming" generally refers to a process of steaming (boiling) using heat treatment, includes aging by feeding moist air at a constant temperature, and is widely used to convert ginseng into red ginseng. Steaming is a heating method for long-term storage and distribution. In oriental medicine, steaming is called "boiling", has been used to reduce toxicity and minimize side effects through heat treatment of herbal medicines to increase therapeutic effectiveness, and is convenient for processing or sectioning.

As used herein, the term "extract" includes an extract obtained by extracting the steamed ginger, a diluted or concentrated liquid of the extract, a dried product obtained by drying the extract, a crude or purified product of the extract, a mixture thereof, and an extract with any formulation that can be formed using the extract. In one embodiment of the present invention, the steamed ginger extract may be in the form of dry powder, but is not limited thereto.

In the steamed ginger extract of the present invention, the method for extracting the steamed ginger is not particularly limited and may be extracted using a method commonly used in the art. Non-limiting examples of the extraction method include hot water extraction, ultrasonic extraction, filtration, and reflux extraction and such a method may be performed alone or in combination of two or more.

In the present invention, the type of extraction solvent used to extract the steamed ginger is not particularly limited and the extraction is performed using a conventional method for extracting natural products known in the art, that is, using a common solvent under conventional conditions of temperature and pressure. For example, in the present invention, the steamed ginger extract is obtained by extraction using a solvent selected from the group consisting of water, C₁-C₄ lower alcohol, n-hexane, ethyl acetate, acetone, acetonitrile, n-butylacetate, 1,3-butylene glycol, methylene chloride and a mixture thereof. In one embodiment of the present invention, extraction may be performed using ethanol as a solvent, but is not limited thereto.

In the present invention, when the steamed ginger is extracted using ethanol as a solvent, the ethanol, is for example, 10% to 100% ethanol, 10% to 90% ethanol, 10% to 80% ethanol, 10% to 70% ethanol, 20% to 90% ethanol, 20% to 80% ethanol, 20% to 70% ethanol, 30% to 90% ethanol, 30% to 80% ethanol, 30% to 70% ethanol, 40% to 90% ethanol, 40% to 80% ethanol, 40% to 70% ethanol, 50% to 90% ethanol, 50% to 80% ethanol, 50% to 70% ethanol, 60% to 90% ethanol, 60% to 80% ethanol, 60% to 70% ethanol, 65% to 75% ethanol, or 70% ethanol, but is not limited thereto.

Then, the prepared extract may be filtered, concentrated, or dried to remove the solvent, and all of filtration, concentration, and drying may be performed as well. For example, filtration may be performed using filter paper or a reduced-pressure filter, concentration may be performed using a reduced pressure concentrator, and drying may be performed using spray drying or freeze-drying, without being limited thereto.

As used herein, the term "degenerative arthritis" refers to a disease in which the articular cartilage surrounding the joint surface of the bone is worn away, thus exposing the bone under the cartilage, inducing inflammation of the synovial membrane around the joint and thus causing pain and deformation, is commonly referred to as "osteoarthritis" and is the most common joint disease.

As used herein, the steamed ginger extract contains 1-dehydro-6-gingerdione, 6-gingerol, 8-gingerol, 10-gingerol, 6-shogaol, 6-paradol, 8-paradol, 1-dehydro-8-gingerdione, a sitologically acceptable salt thereof, and a pharmaceutically acceptable salt thereof, but is not limited thereto.

As used herein, the term "1-dehydro-6-gingerdione" refers to a compound having the molecular formula of C₁₇H₂₂O₄, is a phenolic compound having the structure of the following Formula 1, and has a molecular weight of 290.4 (CAS No. 76060-35-0). In the present invention, the 1-dehydro-6-gingerdione may be isolated from steamed ginger, but is not limited thereto, and may be chemically synthesized using a method known in the art or may be a commercially available material.

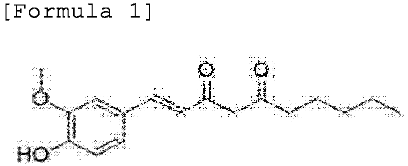

In the present invention, the 1-dehydro-6-gingerdione is a marker component of the steamed ginger extract and the content thereof may be 0.001% by weight or more, for example, 0.001% by weight to 10% by weight, 0.001% by weight to 5% by weight, 0.001% by weight to 3% by weight, 0.001% by weight to 1% by weight, 0.001% by weight to 0.5% by weight, 0.001% by weight to 0.3% by weight, 0.005% by weight to 10% by weight, 0.005% by weight to 5% by weight, 0.005% by weight to 3% by weight, 0.005% by weight to 1% by weight, 0.005% by weight to 0.5% by weight, 0.005% by weight to 0.3% by weight, 0.01% by weight to 10% by weight, 0.01% by weight to 5% by weight, 0.01% by weight to 3% by weight, 0.01% by weight to 1% by weight, 0.01% by weight to 0.5% by weight, 0.01% by weight to 0.3% by weight, 0.1% by weight to 10% by weight, 0.1% by weight to 5% by weight, 0.1% by weight to 3% by weight, 0.1% by weight to 1% by weight, 0.1% by weight to 0.5% by weight, 0.1% by weight to 0.2% by weight, 0.1% by weight to 0.15% by weight, or 0.118% by weight, based on the dry weight of the steamed ginger extract, but is not limited thereto.

The term "marker component" refers to an ingredient determined to control quality among chemically identified ingredients included in raw materials and is a standard for identifying herbal medicines.

As used herein, the term "sitologically acceptable salt" includes salts derived from sitologically acceptable organic acids, inorganic acids, or bases.

As used herein, the term "food" refers to a natural product or processed product containing one or more nutrients, preferably a ready-to-eat food that is already processed, and includes all health functional foods, beverages, food additives, beverage additives and the like.

The food composition used herein may be a health functional food composition, but is not limited thereto.

As used herein, the term "functional food" has the same meaning as food for special health use (FoSHU), refers to processed food with high medical and pharmaceutical effects to efficiently exhibit bioregulatory functions in addition to nutritional supply, and may be prepared as a formulation such as a tablet, capsule, pill, granule, powder, liquid, flake, paste, syrup, gel, jelly, bar, or film. Here, the term "functionality" refers to an effect beneficial for health purposes, such as nutrient regulation or physiological effects in relation to the structure and functions of the human body.

In the present invention, the steamed ginger extract, the 1-dehydro-6-gingerdione isolated therefrom, or the sitologically acceptable salt thereof may be contained as an active ingredient in the food composition for preventing or ameliorating degenerative arthritis.

As used herein, the term "active ingredient" refers to a substance or group of substances that is expected to exhibit directly or indirectly imparts efficacies and effects to preparations based on pharmacological actions of the ingredient itself and the active ingredient is present in an amount of 1% or more by weight, for example, 10% by weight to 100% by weight, 20% by weight to 100% by weight, 30% by weight to 100% by weight, 40% by weight to 100% by weight, 50% by weight to 100% by weight, 60% by weight to 100% by weight, 70% by weight to 100% by weight, 80% by weight to 100% by weight, 90% by weight to 100% by weight, 10% by weight to 80% by weight, 20% by weight to 80% by weight, 30% by weight to 80% by weight, 40% by weight to 80% by weight, 50% by weight to 80% by weight, 60% by weight to 80% by weight, or 70% by weight to 80% by weight, based on the total dry weight of the composition, but is not limited thereto.

When the steamed ginger extract, the 1-dehydro-6-gingerdione isolated therefrom, or the sitologically acceptable salt thereof is added as a food additive, it may be added alone to the food or may be used in conjunction with other foods or food ingredients, and may be suitably used according to conventional methods. The amount of active ingredient that is mixed may be appropriately determined according to the purpose of use (preventive, health-improvement or therapeutic treatment). In general, the steamed ginger extract, the 1-dehydro-6-gingerdione isolated therefrom, or the sitologically acceptable salt thereof may be present in an amount of 15% by weight or less, or 10% by weight or less, based on the raw material. However, in the case of long-term intake for health and hygiene purposes or for health control, the amount may be below the above range, and the active ingredient may be used in an amount above the range, since there is no problem in terms of safety.

There are no particular restrictions on the types of the food. Examples of the food to which the substance can be added include meat, sausage, bread, chocolate, candy, snacks, confectionery, pizza, ramen, other kinds of noodles, gum, dairy products such as ice cream, various soups, beverages, tea, drinks, alcoholic beverages and vitamin complexes, and includes food in a conventional sense.

Like ordinary beverages, the health beverage composition according to the present invention may contain a natural carbohydrate or flavoring agent as an additive. The natural carbohydrate may include monosaccharides such as glucose and fructose, disaccharides such as maltose and sucrose, polysaccharides such as dextrin and cyclodextrin, and sugar alcohols such as xylitol, sorbitol and erythritol. The flavoring agent may include natural flavoring agents such as thaumatin and stevia extracts, and synthetic flavoring agents such as saccharin and aspartame. The content of the natural carbohydrate is typically about 0.01 to 0.20 g, or about 0.04 to 0.10 g with respect to 100 mL of the composition of the present invention.

In addition to the ingredient described above, the composition of the present invention contains various nutrients, vitamins, electrolytes, flavors, colorants, pectic acid and salts thereof, alginic acid and salts thereof, organic acids, protective colloidal thickeners, pH adjusters, stabilizers, preservatives, glycerin, alcohol, carbonating agents used in carbonated drinks and the like. Further, the composition of the present invention may contain pulp for the production of natural fruit juice, fruit juice beverages, and vegetable beverages. These ingredients can be used independently or in combination. The content of these additives is not very important and is generally selected within the range of 0.01 to 0.20 parts by weight with respect to 100 parts by weight of the composition of the present invention.

In another aspect, the present invention provides a pharmaceutical composition for preventing or treating degenerative arthritis containing a steamed ginger extract, 1-dehydro-6-gingerdione isolated therefrom, or a pharmaceutically acceptable salt thereof as an active ingredient.

As used herein, the term "pharmaceutically acceptable salt" includes salts derived from pharmaceutically acceptable inorganic acids, organic acids, or bases.

In another aspect, the present invention provides a method of treating or ameliorating degenerative arthritis including administering a composition containing a steamed ginger extract, 1-dehydro-6-gingerdione isolated therefrom, or a pharmaceutically or sitologically acceptable salt thereof to a subject in need thereof.

In another aspect, the present invention provides the use of a composition containing a steamed ginger extract, 1-dehydro-6-gingerdione isolated therefrom, or a pharmaceutically or sitologically acceptable salt thereof for the prevention, amelioration or treatment of degenerative arthritis.

In another aspect, the present invention provides the use of a composition containing a steamed ginger extract, 1-dehydro-6-gingerdione isolated therefrom, or a pharmaceutically or sitologically acceptable salt thereof for the preparation for drugs for preventing, ameliorating or treating degenerative arthritis.

As used herein, the term "prevention" means any action of preventing the onset of degenerative arthritis or inhibiting progression thereof, as used herein, the term "treatment" refers to any action to alleviate or beneficially change the symptoms of degenerative arthritis, and as used herein, the term "alleviation" means any action that at least reduces the severity of a parameter related to the degenerative arthritis, for example, the degree of a symptom, by administration of the composition of the present invention.

The pharmaceutical composition according to the present invention may further contain an appropriate carrier, excipient, and diluent commonly used in the preparation of pharmaceutical compositions. The excipient may, for example, include at least one selected from the group consisting of diluents, binders, disintegrants, lubricants, adsorbents, humectants, film-coatings, and controlled-release additives.

The pharmaceutical composition according to the present invention may be formulated into a powder, granule, sustained-release granule, enteric-coated granule, solution, eye drop, elixir, emulsion, suspension, spirit, troche, aromatic water, lemonade, tablet, sustained-release tablet, enteric-coated tablet, sublingual tablet, hard capsule, soft capsule, sustained-release capsule, enteric-coated capsule, pill, tincture, soft extract, dry extract, fluid extract, injection, capsule, perfusate, plaster, lotion, paste, spray, inhalant, patch, sterilized injection solution, or a topical agent such as aerosol, and the topical agent may have a formulation such as cream, gel, patch, spray, ointment, plaster, lotion, liniment, pasta, or cataplasm.

The carrier, excipient, and diluent that may be contained in the pharmaceutical composition according to the present invention include lactose, dextrose, sucrose, oligosaccharides, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate and mineral oil.

Upon formulation of the composition, typically used diluents or excipients, such as fillers, extenders, binders, wetting agents, disintegrants or detergents, may be used.

The additives of tablets, powders, granules, capsules, pills, and troches according to the present invention include excipients such as corn starch, potato starch, wheat starch, lactose, white sugar, glucose, fructose, D-mannitol, precipitated calcium carbonate, synthetic aluminum silicate, calcium monohydrogen phosphate, calcium sulfate, sodium chloride, sodium bicarbonate, purified lanolin, microcrystalline cellulose, dextrin, sodium alginate, methylcellulose, sodium carboxymethylcellulose, kaolin, urea, colloidal silica gel, hydroxypropyl starch, hydroxypropylmethyl cellulose (HPMC), HPMC 1928, HPMC 2208, HPMC 2906, HPMC 2910, propylene glycol, casein, calcium lactate, and Primojel^{®}; and binders such as gelatin, gum arabic, ethanol, agar powder, cellulose acetate phthalate, carboxymethyl cellulose, calcium carboxymethyl cellulose, glucose, purified water, sodium caseinate, glycerin, stearic acid, sodium carboxymethyl cellulose, sodium methyl cellulose, methyl cellulose, microcrystalline cellulose, dextrin, hydroxycellulose, hydroxypropyl starch, hydroxymethylcellulose, refined shellac, gelled starch, hydroxypropylcellulose, hydroxypropylmethylcellulose, polyvinyl alcohol, and polyvinylpyrrolidone; disintegrants such as hydroxypropyl methyl cellulose, corn starch, agar powder, methyl cellulose, bentonite, hydroxypropyl starch, sodium carboxymethyl cellulose, sodium alginate, calcium carboxymethyl cellulose, calcium citrate, sodium lauryl sulfate, silicic acid anhydride, 1-hydroxy propyl cellulose, dextran, ion exchange resins, polyvinyl acetate, formaldehyde-treated casein and gelatin, alginic acid, amylose, guar gum, sodium bicarbonate, polyvinylpyrrolidone, calcium phosphate, gelled starch, gum arabic, amylopectin, pectin, sodium polyphosphate, ethyl cellulose, white sugar, magnesium aluminum silicate, di-sorbitol solution, and hard anhydrous silicic acid; and lubricants such as calcium stearate, magnesium stearate, stearic acid, hydrogenated vegetable oil, talc, lycopodium, kaolin, petrolatum, sodium stearate, cacao fat, sodium salicylate, magnesium salicylate, polyethylene glycol (PEG) 4000, PEG 6000, liquid paraffin, Lubri wax, aluminum stearate, zinc stearate, sodium lauryl sulfate, magnesium oxide, Macrogol, synthetic aluminum silicate, silicic anhydride, higher fatty acids, higher alcohol, silicone oil, paraffin oil, polyethylene glycol fatty acid ether, starch, sodium chloride, sodium acetate, sodium oleate, dl-leucine, and hard anhydrous silicic acid.

Additives for the liquid according to the present invention include water, dilute hydrochloric acid, dilute sulfuric acid, sodium citrate, sucrose monostearate, polyoxyethylene sorbitol fatty acid esters (twin esters), polyoxyethylene monoalkyl ethers, lanolin ethers, lanolin esters, acetic acid, hydrochloric acid, aqueous ammonia, ammonium carbonate, potassium hydroxide, sodium hydroxide, prolamin, polyvinylpyrrolidone, ethyl cellulose, sodium carboxymethyl cellulose, and the like.

The syrup according to the present invention may include a solution of white sugar, other sugar, sweetener, or the like and may include a flavoring agent, colorant, preservative, stabilizer, suspending agent, emulsifier, thickener or the like.

The emulsion according to the present invention may include purified water, an emulsifier, preservative, stabilizer, fragrance, or the like.

The suspending agent according to the present invention include acacia, tragacanth, methylcellulose, carboxymethylcellulose, sodium carboxymethylcellulose, microcrystalline cellulose, sodium alginate, hydroxypropyl methylcellulose (HPMC), HPMC 1828, HPMC 2906, HPMC 2910, or the like and may include a surfactant, preservative, stabilizer, colorant, or fragrance, as needed.

The injection according to the present invention may include a solvent such as distilled water for injection, 0.9% sodium chloride injection, IV solution, dextrose injection, dextrose + sodium chloride injection, PEG, lactated IV solution, ethanol, propylene glycol, a combination of non-volatile oil and sesame oil, cottonseed oil, peanut oil, soybean oil, corn oil, ethyl oleate, isopropyl myristic acid, or benzyl benzoate; a solubilizer such as sodium benzoate, sodium salicylate, sodium acetate, urea, urethane, monoethylacetamide, butazolidine, propylene glycol, Tween, nicotinic acid amide, hexamine, and dimethylacetamide; a buffer such as weak acid and a salt thereof (acetic acid and sodium acetate), a weak base and salt thereof (ammonia and ammonium acetate), an organic compound, protein, albumin, peptone, and gum; an isotonic agent such as sodium chloride; a stabilizer such as sodium bisulfite (NaHSO₃) carbon dioxide gas, sodium metabisulfite (Na₂S₂O₅), sodium sulfite (Na₂SO₃), nitrogen gas (N₂), and ethylenediaminetetraacetic acid; a sulfating agent such as sodium bisulfide 0.1%, sodium formaldehyde sulfoxylate, thiourea, disodium ethylenediaminetetraacetate, and acetone sodium bisulfite; an analgesic such as benzyl alcohol, chlorobutanol, procaine hydrochloride, glucose, or calcium gluconate; and a suspending agent such as SiMCsodium, sodium alginate, Tween 80, or aluminum monostearate.

As a suppository according to the present disclosure, a base such as cacao butter, lanolin, Witepsol, polyethylene glycol, glycerogelatin, methyl cellulose, carboxymethyl cellulose, a mixture of stearic acid and oleic acid, subanal, cottonseed oil, peanut oil, palm oil, cacao butter+cholesterol, lecithin, lanette wax, glycerol monostearate, tween or span, imhausen, monolen (propylene glycol monostearate), glycerin, adeps solidus, Buytyrum Tego-G, cebes pharma 16, hexalide base 95, cotomar, Hydrokote SP, S-70-XXA, S-70-XX75 (S-70-XX95), hydrokote 25, hydrokote 711, idropostal, massa estrarium (A, AS, B, C, D, E, I, T), martha-MF, marsupol, marsupol-15, Neosupostal-N, paramound-B, suposiro (OSI, OSIX, A, B, C, D, H, L), suppository type IV (AB, B, A, BC, BBG, E, BGF, C, D, 299), Supostal (N, Es), Wecobi (W, R, S, M, Fs), and testester triglyceride base (TG-95, MA, 57) may be used.

Examples of solid formulations for oral administration may include tablets, pills, powders, granules, capsules, and the like, and these solid formulations may be prepared by mixing the extract with at least one excipient such as starch, calcium carbonate, sucrose or lactose, and gelatin. In addition to simple excipients, lubricants such as magnesium stearate and talc may also be used.

Examples of liquid formulations for oral administration may include suspensions, internal solutions, emulsions, syrups, and the like, and various excipients such as wetting agents, sweeteners, flavoring agents, and preservatives may be included in addition to water and liquid paraffin, which are commonly used simple diluents. Examples of formulations for parenteral administration may include sterilized aqueous solutions, non-aqueous solvents, suspensions, emulsions, freeze-dried formulations, and suppositories. Propylene glycol, polyethylene glycol, vegetable oils such as olive oil, injectable esters such as ethyl oleate, and the like may be used as non-aqueous solvents and suspending agents.

The pharmaceutical composition according to the present invention may be administered in a pharmaceutically effective amount. In the present disclosure, a "pharmaceutically effective amount" means an amount sufficient to treat a disease with a reasonable benefit/risk ratio applicable to medical treatment, and the effective amount may be determined according to the type of disease, the severity of disease, the activity of drug, the sensitivity to drug, the time of administration, the route of administration and the excretion rate, the duration of treatment, factors including drugs used concurrently, and other factors well known in medicine.

The pharmaceutical composition according to the present invention may be administered as an individual therapeutic agent or in combination with other therapeutic agents, and may be administered sequentially or concurrently with an existing therapeutic agent and once or multiple times. It is important to administer the pharmaceutical composition according to the present invention in an amount to obtain the maximum effect with the minimum amount without side effects in consideration of all the above-mentioned factors, and such amount may be easily determined by those skilled in the technical field to which the present invention pertains.

The pharmaceutical composition according to the present invention may be administered to a subject by various routes. All administration methods are expectable and may include oral administration, subcutaneous injection, intraperitoneal administration, intravenous injection, intramuscular injection, injection in the paraspinal space (intrathecal), sublingual administration, buccal administration, insertion into the rectum, insertion into the vagina, intraocular administration, ear administration, nasal administration, inhalation, spraying through the mouth or nose, dermal administration, transdermal administration, and the like.

The pharmaceutical composition according to the present invention may be determined according to the type of drug as an active ingredient along with various related factors such as the type of disease to be treated, the route of administration, the patient's age, the patient's gender, the patient's weight, and the severity of disease.

The steamed ginger extract, the 1-dehydro-6-gingerdione isolated therefrom, or the pharmaceutically acceptable salt thereof may be administered in a daily dose of 200 to 600 mg, 250 to 600 mg, 300 to 600 mg, 350 to 600 mg, 400 to 600 mg, 450 to 600 mg, 200 to 550 mg, 200 to 500 mg, 250 to 550 mg, 250 to 500 mg, 300 to 550 mg, 300 to 500 mg, 350 to 550 mg, 350 to 500 mg, 400 to 550 mg, 400 to 500 mg, 450 to 550 mg, 450 to 500 mg, or 480 mg for 6 to 18 weeks, 6 to 16 weeks, 6 to 14 weeks, 8 to 18 weeks, 8 to 16 weeks, 8 to 14 weeks, 10 to 18 weeks, 10 to 16 weeks, 10 to 14 weeks, or 12 weeks, without being limited thereto.

As used herein, the term "subject" means a subject in need of treatment of a disease, and, more specifically, a mammal such as a human or non-human primate, mouse, rat, dog, cat, horse, and cow.

As used herein, the term "administration" means providing the predetermined composition according to the present invention to a subject by any suitable method.

In another aspect, the present invention provides a method for preparing a composition for preventing, ameliorating, or treating degenerative arthritis containing a steamed ginger extract, the method including:
(a) washing ginger and steaming the ginger at 85 to 105°C for 1 to 3 hours;
(b) drying the steamed ginger at 40 to 60°C for 30 to 50 hours;
(c) extracting the dried steamed ginger in a solvent at 75 to 95°C for 10 to 20 hours;
(d) filtering the extracted steamed ginger, followed by concentration under reduced pressure at a temperature of 10 to 65°C; and
(e) filtering the steamed ginger extract to prepare a steamed ginger extract powder.

In the present invention, the steaming in step (a) is performed at 85 to 105°C, 90 to 105°C, 95 to 100°C, or 97°C for 1 to 3 hours, 1 to 2.5 hours, 1.5 to 3 hours, 1.5 to 3 hours, 2.5 hours, or 2 hours, but is not limited thereto.

In the present invention, the drying in step (b) may be performed for more than 30 hours, for example, at 40 to 60°C, 45 to 60°C, 45 to 55°C, or 50°C for 30 to 50 hours, 30 to 45°C, 30 to 40 hours, or 30 to 35 hours, but is not limited thereto.

In the present invention, the extraction in step (c) may be performed at 75 to 95°C, 75 to 90°C, 80 to 95°C, 80 to 90°C, or 85°C for 10 to 20 hours, 10 to 17 hours, 12 to 2 hours, 12 to 17 hours, or 15 hours, but is not limited thereto.

In the present invention, the concentration under reduced pressure in step (d) may be performed at a temperature of 65°C or lower, for example, 10 to 65°C, 20 to 65°C, 30 to 65°C, 40 to 65°C, 50 to 65°C, or 60 to 65°C, to 20 brix or more, for example, 20 to 100 brix, 20 to 80 brix, 20 to 60 brix, or 20 to 40 brix, but is not limited thereto.

In the present invention, step (e) may further include filtering the steamed ginger extract concentrated under reduced pressure, and mixing the steamed ginger extract with carbohydrates at a dry weight ratio of 1:0.5 to 1:2, 1:0.5 to 1:1.5, 1:0.5 to 1:1, 1:1 to 1:2, 1:1 to 1:1.5, or 1:1. In one embodiment of the present invention, the carbohydrate may be dextrin, but is not limited thereto.

In the present invention, step (e) may further include sterilizing the mixture of the steamed ginger extract and carbohydrates at 80 to 100°C, 85 to 100°C, 80 to 95°C, 85 to 95°C, or 90°C for 10 to 20 minutes, 10 to 17 minutes, 12 to 17 minutes, or 15 minutes, but is not limited thereto.

In the present invention, the spray drying in step (e) may be performed in a dryer having an inlet temperature of 180 to 200°C, 180 to 195°C, 185 to 200°C, 185 to 195°C, or 190°C, and an outlet temperature of 80 to 100°C, 80 to 95°C, 85 to 100°C, 85 to 95°C, or 90°C, but is not limited thereto.

Hereinafter, the present invention will be described in more detail with reference to preferred examples and experimental examples. However, the following examples and experimental examples are provided only for illustration and should not be construed as limiting the scope of the present invention.

### [Example]

### Example 1. Preparation of steamed ginger extract

### 1-1. Steamed ginger preparation

Fresh ginger was purchased and the rhizome thereof was used. The rhizome was washed with purified water for 5 to 7 minutes and the washed ginger was steamed in a steamer at 97°C for 2 hours. Then, the steamed ginger was dried in a dryer at 50°C for 30 hours or more to prepare steamed ginger.

### 1-2. Preparation of steamed ginger extract

70% alcohol (ethanol) was added in an amount of 15 times to the steamed ginger raw material prepared in Example 1-1, followed by extraction at 85°C for 15 hours. Then, the filtrate was concentrated under reduced pressure to 20 brix or more at a low temperature of 65°C or less and filtered again. Then, the steamed ginger concentrate was mixed with dextrin, followed by sterilization at 90°C for 15 minutes, and then spray-drying in a dryer with an inlet temperature of 190°C and an outlet temperature of 90°C to prepare a steamed ginger extract powder.

The preparation process and conditions of the steamed ginger extract powder as described above are shown in Table 1 below.

**[Table 1]**

| (1) Preparation process | (2) Process, food, food additive | (3) Change in function/marker component content (mg/g) | (4) Yield (kg) |
|---|---|---|---|
| Raw materials | Steamed ginger | 4.656 | 65 |
| ↓ | | | |
| Extraction | 15x 70% alcohol, 85°C for 15 hours | 2.156 | 970 |
| ↓ | | | |
| Filtration | - | 2.073 | 970 |
| ↓ | | | |
| Concentration | concentrated to 20 brix or more at low temperature (65°C or less) | 2.111 | 35 |
| ↓ | | | |
| Filtration | - | 2.130 | 35 |
| ↓ | | | |
| Mixing | Extract (solid 100) 50%: dextrin 50% | 1.147 | 42 |
| ↓ | | | |
| Sterilization | at 90°C for 15 minutes | 1.147 | 42 |
| ↓ | | | |
| Freeze-drying | Inlet 190°C, outlet 90°C | 1.140 | 11.3 |
| ↓ | | | |
| Packaging | Packaging | 1.140 | 11.3 |

### Example 2. Isolation of compounds from steamed ginger extract

Solvent systematic fractionation was performed using n-hexane and distilled water from the steamed ginger extract prepared in Example 1. The main components such as 1-dehydro-6-gingerdione, 6-gingerol, 8-gingerol, 10-gingerol, 6-shogaol, 6-paradol, 8-paradol), and 1-dehydro-8-gingerdione were obtained from the obtained n-hexane fraction by repeated open column chromatography using various resins such as SiO₂, ODS, and Sephadex LH-20.

In addition, liquid chromatography (high pressure liquid chromatography (HPLC) analysis was performed on the steamed ginger extract under the following conditions.

The steamed ginger extract and ginger extract were dissolved in 80% methanol to prepare a 10,000 ppm solution, and then the solution was purified through a 0.22 µm membrane filter (Woongki Science Co., Ltd., Seoul, Korea) to prepare a sample. The HPLC used for analysis was the Agilent Technologies 1260 series, and Cadenza CD-C18 (3 µm, 250 × 4.6 mm) was used as the analytical column. At this time, the wavelength for measurement was 370 nm, the volume of sample injected was 10 µL, the solvent elution rate was 0.4 mL/min, the solvent for analysis was a mixture of water containing 0.1% formic acid (A) and acetonitrile (B), and observations were conducted for 60 minutes while changing the composition of the solvent as follows.

Based on (B), 30% (0 min) → 30% (5 min) → 55% (10 min) → 55% (13 min) -. 80% (20 min) → 80% (23 min) → 100% (30 min) → 100% (60 min) .

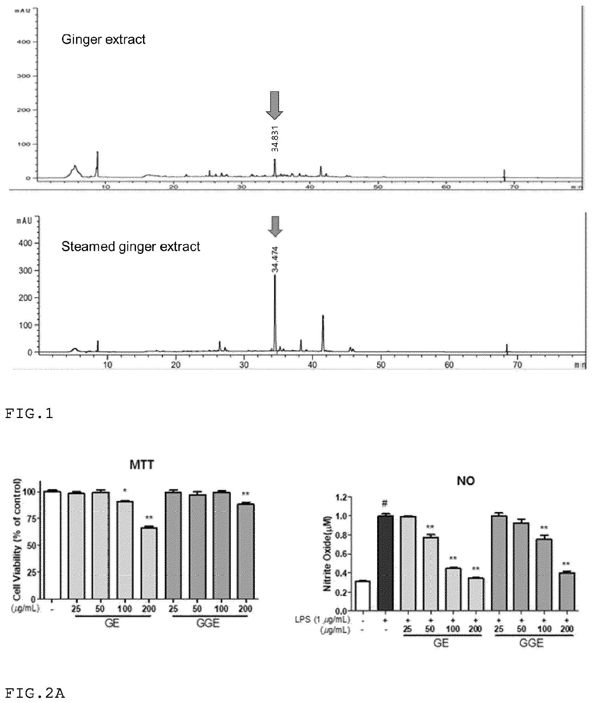

As a result, when comparing the steamed ginger extract with the ginger extract, as can be seen from FIG. 1, the content of 1-dehydro-6-gingerdione was significantly increased. Therefore, 1-dehydro-6-gingerdione was set as a marker component. In order to detect the content of 1-dehydro-6-gingerdione, a marker component in the steamed ginger, a calibration curve was plotted using a standard product. It can be seen from the plotted calibration curve that 1-dehydro-6-gingerdione was present in an amount of 1.18 mg/g. The content of 1-dehydro-6-gingerdione in the ginger extract was 0.54 mg/g.

### [Experimental example]

### Experimental Example 1. Confirmation of anti-inflammatory effect in vitro

### 1-1. Confirmation of cell viability and NO production inhibition effect

The cell viability and NO production inhibition effect of active ingredients of the ginger extract (GE), steamed ginger extract (GGE), and steamed ginger were detected in RAW 264.7 cells, which are mouse macrophages.

Cell viability was confirmed using MTT (3-(4,5-dimethylthiazol-2-yl)-2,5-diphenyltetrazolium bromide). The absorbance was measured using the characteristic that viable cells convert MTT into formazan. RAW 264.7 cells were inoculated in a 96 well-plate at a concentration of 1 × 10⁴ cells/well, incubated in an incubator at 37°C and 5% CO₂ for 24 hours, treated with the ginger extract (GE) and the steamed ginger extract (GGE) at concentrations of 25, 50, 100, and 200 µg/mL, treated with the active ingredients of the steamed ginger in concentrations of 1.3, 2.5, 5, 10 and 20 µg/mL, incubated for 24 hours, supplemented with 10 µL of 5 mg/mL MTT solution and then incubated for 2 hours. Then, the medium supernatant was removed and dissolved in 100 µL of DMSO, and the absorbance was measured at 540 nm.

To confirm the NO production inhibition effect, RAW 264.7 cells were inoculated into a 96-well plate at a concentration of 1×10⁴ cells/well, incubated in an incubator at 37°C and 5% CO₂ for 24 hours, pretreated with LPS (lipopolysaccharide) at a concentration of 1 ug/mL for 2 hours to induce inflammation, treated with the steamed ginger extract at concentrations of 25, 50, 100, and 200 pg/mL, treated with the active ingredients of steamed ginger at concentrations of 1.3, 2.5, 5, 10 and 20 µg/mL, and incubated for 24 hours. Then, 50 µL of the supernatant was transferred to a fresh 96-well plate, supplemented with 50 µL of Griess reagent, and reacted at room temperature for 20 minutes and absorbance was measured at 450 nm.

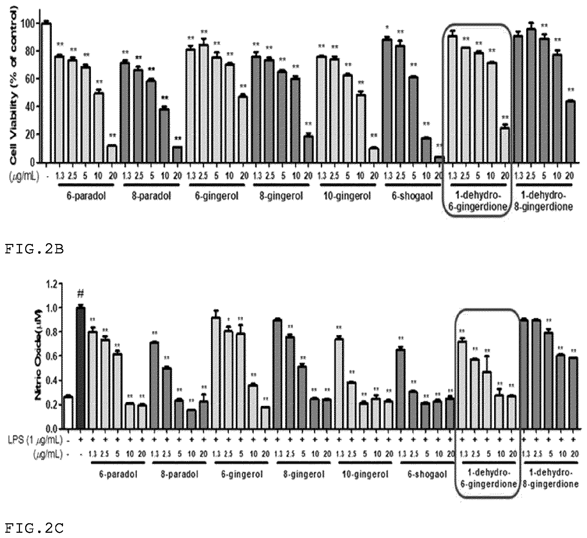

As a result, as can be seen from FIG. 2A, the steamed ginger extract had lower cytotoxicity than the ginger extract (left of FIG. 2) and exhibited an effect of inhibiting NO production induced by LPS (right of FIG. 2).

In addition, as can be seen from FIGS. 2B and 2C, among the active ingredients of the steamed ginger, the indicator component, 1-dehydro-6-gingerdione, exhibits low cytotoxicity (FIG. 2B) and has high NO production inhibitory effect in a concentration-dependent manner (FIG. 2C).

### 1-2. Confirmation of IL-1β expression inhibition effect

mRNA and protein expression of interleukin-1 beta (IL-1β) in RAW 264.7 cells upon treatment with 1-dehydro-6-gingerdione (6-GD), an active ingredient of the steamed ginger extract (GGE) and the steamed ginger was confirmed.

Specifically, RAW 264.7 cells were seeded at 1×10⁴ cells/well in a 6-well plate, pretreated with LPS at a concentration of 1 µg/mL for 2 hours, and treated with the steamed ginger extract at different concentrations (50, 100, and 200 pg/mL) for 24 hours. Total RNA was extracted using TRIzol solution, RNA was quantified using Nanodrop, and cDNA was synthesized from an equal amount of RNA using a cDNA synthesis kit (Takara, Japan), followed by qRT-PCR (quantitative real time-PCR).

Primer sequences used in the experiment are shown in Table 2 below.

**[Table 2]**

| Gene | Primer sequences | SEQ. ID NO. |
|---|---|---|
| IL-1β | 5'-GCAACTGTTCCTGAACTCAACT-3' (sense) | 1 |
| | 5'-ATCTTTTGGGGTCCGTCAACT-3' (antisense) | 2 |
| β-actin | 5'-CGTGAAAAGATGACCAGAT-3' (sense) | 3 |
| | 5'-ACCCTCATAGATGGCACA-3' (antisense) | 4 |

In addition, to identify the protein expression of interleukin-1 beta (IL-1β), the protein was extracted using RIPA buffer, and an equal amount of protein was electrophoresed using 12% SDS-PAGE and then transferred to a nitrocellulose membrane. Then, the result was blocked with 5% skim milk at room temperature for 1 hour, followed by reaction with a primary antibody against IL-1β (Invirogen, USA) at 4°C for one day, and then reacted with a secondary antibody conjugated with an enzyme, radioactive substance, or fluorescent substance (HPR-conjugated rabbit, Cell Signaling, USA) at room temperature for 1 hour. After 1 hour, the protein was detected using Lumi-Pico reagent.

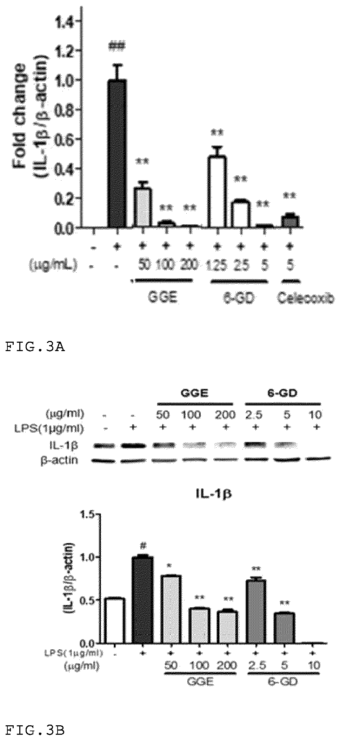

As a result, as can be seen from FIGS. 3A and 3B, the expression of the mRNA (FIG. 3A) and protein (FIG. 3B) of IL-1β, an inflammatory cytokine, was decreased by treatment with the steamed ginger extract or 1-dehydro-6-gingerdione.

### Experimental Example 2. Confirmation of effect in degenerative arthritis animal model

### 2-1. Degenerative arthritis animal model induction and steamed ginger extract administration

50 µL (3 mg/kg) of MIA (mono-iodoacetate) was administered to the joint cavity of 5-week-old Wistar rats for 2 weeks to induce degenerative arthritis.

Then, the ginger extract (200 mg/kg), the steamed ginger extract (50, 100, or 200 mg/kg), and celecoxib (10 mg/kg) as a positive control (PC) were orally administered for 6 weeks, and micro-CT analysis, joint structure and inflammatory marker analysis were performed in blood and articular cartilage.

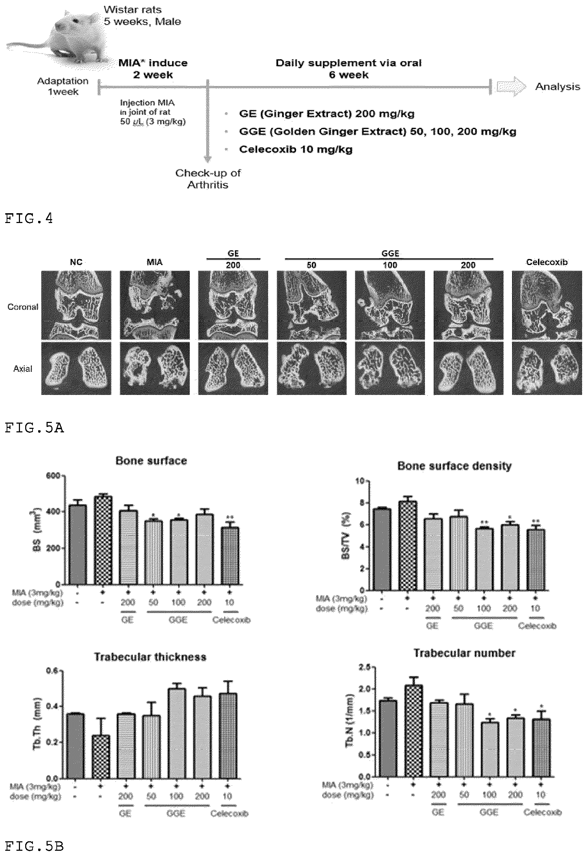

The schematic diagram illustrating the process of inducing the degenerative arthritis animal model and administering the steamed ginger extract as described above is shown in FIG. 4.

### 2-2. Micro-CT analysis

Micro-CT imaging and analysis were performed to identify structural changes in joint tissue using the MIA-induced degenerative arthritis animal model of Experimental Example 2-1.

As a result, as can be seen from FIG. 5A, the MIA-induced group had degenerative arthritis due to destroyed cartilage tissue, compared to the normal group (NC), whereas the ginger extract (GE)-, steamed ginger extract (GGE)-, and PC (celecoxib)-administered groups had restored cartilage tissue to a level comparable to the normal group.

In addition, as can be seen from FIG. 5B, bone surface area (BS), bone surface density (BS/TV), and trabecular number (Tb.N) were increased by MIA, but were decreased by administration of the ginger extract and steamed ginger extract. The trabecular thickness (Tb.Th) was decreased by MIA, but was increased by administration of the ginger extract and the steamed ginger extract. In particular, it was found that the steamed ginger extract-administered group had a greater cartilage tissue restoration effect compared to the ginger extract-administered group.

### 2-3. Confirmation of inflammatory cytokine expression in blood of degenerative arthritis animal model

Expression of inflammatory cytokines was identified in the blood of the MIA-induced degenerative arthritis animal model of Experimental Example 2-1.

Specifically, the ginger extract and steamed ginger extract were administered to animals with MIA-induced degenerative arthritis for 6 weeks and fasted before autopsy, blood was collected from the abdominal vein of the animals, and the serum was separated by centrifugation at 4°C and 12,000 rpm for 15 minutes. The contents of IL-1β, TNF-α, and PGE 2 were measured after reaction with the items included in the kit (R&D system, USA) in accordance with the manufacturer's test method in order to measure the content of inflammatory cytokines in the blood.

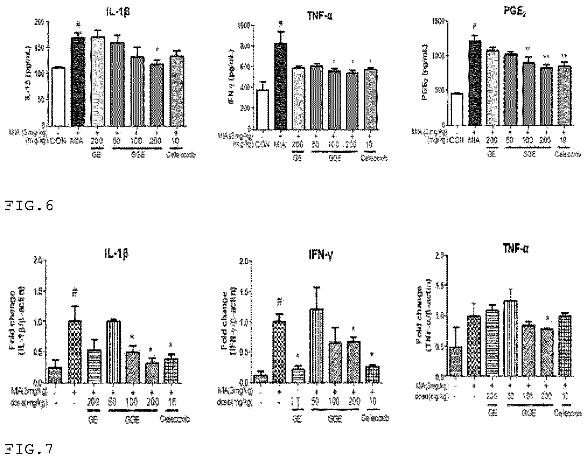

As a result, as can be seen from FIG. 6, the expression of inflammatory cytokines, IL-1β, TNF-α, and prostaglandin E₂ (PGE₂) in the blood of the MIA-induced degenerative arthritis animal model was significantly decreased by treatment with the steamed ginger extract. It can be seen that the effect of reducing the expression of inflammatory cytokines was higher when treated with the steamed ginger extract compared to when treated with the ginger extract.

### 2-4. Confirmation of inflammatory cytokine expression in cartilage of degenerative arthritis animal model

Expression of inflammatory cytokines was identified in the cartilage of the MIA-induced degenerative arthritis animal model of Experimental Example 2-1.

Specifically, knee joint cartilage tissue was collected from experimental animals, RNA was extracted using TRIzol reagent, RNA was assayed using Nanodrop, cDNA was synthesized from an equal amount of RNA using a cDNA synthesis kit (Takara, Japan) and then qRT-PCR was performed.

Primer sequences used in the experiment are shown in Table 3 below.

**[Table 3]**

| Gene | Primer sequences | SEQ. ID NO. |
|---|---|---|
| IL-1β | 5'-CACCTCTCAAGCAGAGCACAGCACCTC-3' (sense) | 5 |
| | 5'-GGGTTCCATGGTGAAGTCAAC-3' (antisense) | 6 |
| TNF-α | 5'-TCGTCTACTACTCCTCAGAGCCC-3' (sense) | 7 |
| | 5'-ACTTCAGCGTCTCGTGTGTT-3' (antisense) | 8 |
| IFN-γ | 5'-GCAAAGGACGGTAACACGAA-3' (sense) | 9 |
| | 5'-ATGGCCTGGTTGTTGTCTTTCAAGA-3' (antisense) | 10 |
| β-actin | 5'-CGTGAAAAGATGACCCAGAT-3' (sense) | 11 |
| | 5'-ACCCTCATAGATGGGCACA-3' (antisense) | 12 |

As a result, as can be seen from FIG. 7, the expression of inflammatory cytokines IL-1β, IFN-γ, and TNF-α in the cartilage of the MIA-induced degenerative arthritis animal model was significantly reduced by treatment with the steamed ginger extract. In particular, the effect of inhibiting the expression of IL-1β and TNF-α was higher when treated with the steamed ginger extract compared to when treated with the ginger extract.

### 2-5. Confirmation of cartilage decomposition inhibition and production effects in cartilage of degenerative arthritis animal model

The expressions of MMPs (matrix metalloproteinases) that decompose cartilage components such as collagen and gelatin in the cartilage of an MIA-induced degenerative arthritis animal model, TIMPs (issue inhibitor of metalloproteinases) that inhibit the action of the MMPs, and COL-2 (collagen type II) that is involved in the creation of cartilage tissue were confirmed.

Specifically, in order to confirm the effects of inhibiting cartilage decomposition and creating cartilage in the articular cartilage of the animal model of degenerative arthritis, RNA was extracted from the cartilage tissue of the experimental animal using the TRIzol reagent in the same manner as in Experimental Example 2-4, RNA was assayed with Nanodrop, cDNA was synthesized from an equal amount of RNA using a cDNA synthesis kit (Takara, Japan), and then gene expression was identified using qRT-PCR.

Primer sequences used in the experiment are shown in Table 4 below.

**[Table 4]**

| Gene | Primer sequences | SEQ. ID NO. |
|---|---|---|
| MMP-9 | 5'-AGCCGGGAACGTATCTGGA-3' (sense) | 13 |
| | 5'-TGGAAACTCACACGCCAGAAG-3' (antisense) | 14 |
| TIMP-1 | 5'-CATCTCTGGCCTCTGGCATC-3' (sense) | 15 |
| | 5'-CATAACGCTGGTATAAGGTGGTCTC-3' (antisense) | 16 |
| TIMP-2 | 5'-CTGTGACCCAGTCCATCCAGAG-3' (sense) | 17 |
| | 5'-GACACGCTTAGCATCACCCAGA-3' (antisense) | 18 |
| COL2 | 5'-CTGAGGGCTCCCAGAACATC-3' (sense) | 19 |
| | 5'-TTCGTCCAGGTAGGCAATGC-3' (antisense) | 20 |
| β-actin | 5'-CGTGAAAAGATGACCCAGAT-3' (sense) | 11 |
| | 5'-ACCCTCATAGATGGGCACA-3' (antisense) | 12 |

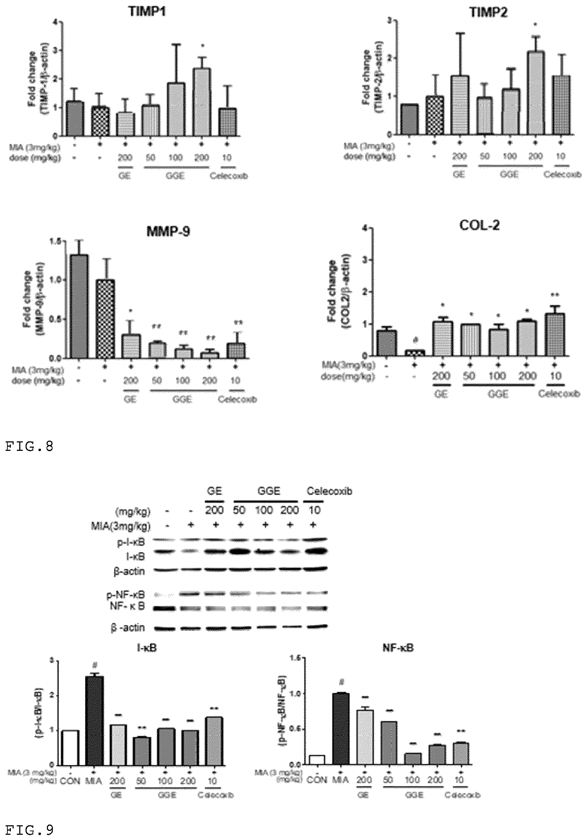

As a result, as can be seen from FIG. 8, when the cartilage of the animal model of MIA-induced degenerative arthritis was treated with the steamed ginger extract compared to when treated with the ginger extract, the expression of TIMP1 and TIMP2 was increased, the expression of MMP-9 was decreased, and the expression of COL-2 was significantly increased.

This indicates that the steamed ginger extract according to the present invention has effects of inhibiting cartilage decomposition and creating cartilage in degenerative arthritis.

### 2-6. Confirmation of I-xB and NF-κB expression in cartilage of degenerative arthritis animal model

The expression of I-κB and NF-κB proteins, which are involved in the production of inflammatory cytokines and MMPs, was identified in the cartilage of the MIA-induced degenerative arthritis animal model as the degenerative arthritis progresses.

Specifically, to identify the expression of IκB and NF-κB, proteins were extracted from cartilage tissue of experimental animals using RIPA buffer, and an equal amount of protein was electrophoresed using 12% SDS-PAGE and then transferred to a nitrocellulose membrane. Then, the result was blocked with 5% skim milk at room temperature for 1 hour, and then reacted with primary antibodies against phospho-IκB (Santa Cruz, USA), IκB (Santa Cruz, USA), phospho-NF-κB (Cell Signaling, USA), and NF-κB (Cell Signaling, USA) at 4°C for one day, and then reacted with a secondary antibody conjugated with an enzyme, radioactive substance, or fluorescent substance (HPR-conjugated rabbit or mouse, Cell Signaling, USA) at room temperature for 1 hour. After 1 hour, proteins were detected and assayed using Lumi-Pico reagent.

As a result, as can be seen from FIG. 9, the protein expression of I-κB and NF-κB was significantly reduced in the cartilage of the MIA-induced degenerative arthritis animal model, when treated with the steamed ginger extract compared to when treated with the ginger extract. This indicates that the steamed ginger extract according to the present invention had an effect of ameliorating degenerative arthritis due to the anti-inflammatory effect resulting from reduced NF-κB.

Although embodiments of the present invention have been described above, it will be obvious to those skilled in the art that the present invention can be implemented in other specific embodiments without changing the technical concepts or essential features of the present invention. Therefore, it should be construed that the aforementioned embodiments are illustrative and not restrictive in all respects.

### [Industrial applicability]

The steamed ginger extract or the 1-dehydro-6-gingerdione isolated therefrom according to the present invention is expected to be useful for functional food compositions, pharmaceutical compositions, and the like for preventing, ameliorating, or treating degenerative arthritis, and thus has industrial applicability.

## Claims

1. A food composition for preventing or ameliorating degenerative arthritis comprising a steamed ginger extract, 1-dehydro-6-gingerdione isolated therefrom, or a sitologically acceptable salt thereof as an active ingredient.

2. The method according to claim 1, wherein the food composition is a functional food composition.

3. The method according to claim 1, wherein the steamed ginger extract is obtained by extraction using a solvent selected from the group consisting of water, C₁-C₄ lower alcohol, n-hexane, ethyl acetate, acetone, acetonitrile, n-butylacetate, 1,3-butylene glycol, methylene chloride and a mixture thereof.

4. The method according to claim 1, wherein the steamed ginger extract comprises at least one selected from the group consisting of 1-dehydro-6-gingerdione, 6-gingerol, 8-gingerol, 10-gingerol, 6-shogaol, 6-paradol, 8-paradol, 1-dehydro-8-gingerdione, a sitologically acceptable salt thereof, and a pharmaceutically acceptable salt thereof.

5. The method according to claim 4, wherein the steamed ginger extract is present in an amount of 0.001% to 10% by weight based on a dry weight of the steamed ginger extract.

6. The method according to claim 1, wherein the composition reduces expression of at least one selected from the group consisting of IL-1β, TNF-α, IFN-γ, and prostaglandin E₂ (PGE₂).

7. The method according to claim 1, wherein the composition has an activity of restoring cartilage tissue, creating cartilage, or inhibiting cartilage decomposition.

8. The method according to claim 1, wherein the composition reduces expression of I-xB or nuclear factor (NF)-xB protein.

9. A pharmaceutical composition for preventing or treating degenerative arthritis comprising a steamed ginger extract, 1-dehydro-6-gingerdione isolated therefrom, or a pharmaceutically acceptable salt thereof as an active ingredient.

10. The pharmaceutical composition according to claim 9, wherein the steamed ginger extract comprises at least one selected from the group consisting of 1-dehydro-6-gingerdione, 6-gingerol, 8-gingerol, 10-gingerol, 6-shogaol, 6-paradol, 8-paradol, 1-dehydro-8-gingerdione, a sitologically acceptable salt thereof, and a pharmaceutically acceptable salt thereof.

11. A method for preparing a composition for preventing, ameliorating, or treating degenerative arthritis comprising a steamed ginger extract, the method comprising:
(a) washing ginger and steaming the ginger at 85 to 105°C for 1 to 3 hours;
(b) drying the steamed ginger at 40 to 60°C for 30 to 50 hours;
(c) extracting the dried steamed ginger in a solvent at 75 to 95°C for 10 to 20 hours;
(d) filtering the extracted steamed ginger, followed by concentration under reduced pressure at a temperature of 10 to 65°C; and
(e) filtering the steamed ginger extract to prepare a steamed ginger extract powder.

12. A method of treating or ameliorating degenerative arthritis comprising administering a composition comprising a steamed ginger extract, 1-dehydro-6-gingerdione isolated therefrom, or a pharmaceutically or sitologically acceptable salt thereof to a subject in need thereof.

13. Use of a composition comprising a steamed ginger extract, 1-dehydro-6-gingerdione isolated therefrom, or a pharmaceutically or sitologically acceptable salt thereof for prevention, amelioration or treatment of degenerative arthritis.

14. Use of a composition comprising a steamed ginger extract, 1-dehydro-6-gingerdione isolated therefrom, or a pharmaceutically or sitologically acceptable salt thereof for preparation for drugs for preventing, ameliorating or treating degenerative arthritis.
